# EUROPEAN PATENT APPLICATION

(11) **EP 2 267 119 A1**
(43) Date of publication of application: **29.12.2010**
(21) Application number: 09727365.0
(22) Date of filing: 31.03.2009
(51) Int. Cl.: C12N 7/02, C12N 15/09

(54) **METHOD FOR CONCENTRATION OF VIRUS**

(30) Priority: 31.03.2008 JP 2008092816
(71) Applicant: Japan Tobacco, Inc., Tokyo 105-8422 (JP)
(72) Inventor: TAKAKURA, Yoshimitsu, Iwata-shi Shizuoka 438-0802 (JP); ICHIKAWA, Masako, Iwata-shi Shizuoka 438-0802 (JP); KONDO, Kazuhiro, Tokyo 105-8461 (JP); SHIMIZU, Akihiro, Tokyo 152-0032 (JP)
(74) Representative: Behnisch, Werner
(86) International application number: PCT/JP2009/057040
(87) International publication number: WO 2009/123348

(57) **Abstract**

The present invention provides a novel method that can increase readily a virus or viral vector concentration in a solution having a low concentration and a kit for performing the method. Conventional methods require complicated operations, expensive equipment, or highly trained experts for efficiently concentrating viruses from low-concentration virus solutions. The method of the present invention can concentrate viral vectors readily while maintaining infection abilities of the viral vectors, and thus it can be used as a safe and simple technique for concentrating a vector useful in the field of a genetic therapy or a vaccine therapy using a viral vector.

## Description

### TECHNICAL FIELD

The present invention relates to a novel method of increasing the concentration of a virus or a viral vector while maintaining an infection ability of the same, and a kit for performing the method. The present invention relates to a safe and simple technique for increasing a concentration of a vector such as a retroviral vector or a herpesviral vector useful in the field of a genetic therapy, vaccines, and the like.

### BACKGROUND ART

Recently, various viral vectors have been developed for genetic therapies and vaccine therapies, and their positive results are expected. However, since desirable effects cannot be sufficiently obtained at a low concentration of a viral vector, a concentration is required to be adjusted to an adequate level. In the field of genetic therapy, the success of therapy depends on the efficiency of gene introduction into cells, and therefore viral vectors with high gene introduction efficiency are used. However, regarding retroviral vectors including lentiviral vectors, viruses showing high titers have not been obtained; hence, the efficiency of gene introduction is not sufficient. Accordingly, various processes for concentrating viruses and products based on such processes have been developed, but they still have various practical problems, as the matter stands.

A method of raising the concentration of a virus in a solution and thereby concentrating the virus is, for example, ultracentrifugation. Unfortunately, this method requires use of expensive equipment and a long time for separation and thus a large amount of work is required to carry out the method. Another known method of concentrating a virus is precipitation of the virus with ammonium sulfate or polyethylene glycol. Unfortunately, such a method may preclude the subsequent procedures because the reagent used and other precipitated components in the method are harmful to infected cells. This requires purification of the sample after precipitation of the virus, which is disadvantageous in that it requires a large amount of work. A method of concentrating virus particles themselves by using an antibody has also been developed (Japanese Patent Public Disclosure No. 2002-78485). However, this method may inhibit the function of protein on the virus surface and thereby affect infection ability of the virus. For example, virus particles should be separated from an antibody under strongly acidic conditions (for example, a pH of 2 to 3). This may cause defects in the virus particles.

Another disclosed method of concentrating a virus is, for example, use of magnetic particles to which a mannose-binding lectin is immobilized (Japanese Patent Public Disclosure No. 2002-165591).

In addition, disclosed is a method using streptavidin and a biotinylated lectin that enhances the retroviral titer or isolates a retrovirus from a sample (W02001/079456). In all the disclosures described above, the viruses used are bound to carriers, and the virus particles are not isolated. Accordingly, applications of the viruses concentrated by these methods are limited.

### PRIOR ART DOCUMENTS

Patent Document 1: Japanese Unexamined Patent Application Publication No. 2002-078485
Patent Document 2: Japanese Unexamined Patent Application Publication No. 2002-165591
Patent Document 3: International Publication No. WO2001/079456

### DISCLOSURE OF THE INVENTION

### PROBLEMS TO BE SOLVED BY THE INVNETION

A concentration of a virus in a body fluid cannot be determined quantitatively at a level lower than a detection limit of a known method. If a concentration of a viral vector that is used in, for example, a genetic therapy or a vaccine therapy is low, a desirable therapeutic effect cannot be obtained. Thus, adjustment of the concentration is required.

Accordingly, it is an object of the present invention to provide a simple method of concentrating a virus or a viral vector in a solution. Another object of the present invention is to provide a simple method of concentrating a virus or a viral vector from a low level virus solution and obtaining the virus or the viral vector in an isolated state maintaining infection ability. In particular, it is an object of the present invention to provide a safe and simple technique of increasing a concentration of a vector such as a retroviral vector or a herpesviral vector useful for genetic therapy or a vaccine.

### MEANS FOR SOLVING THE PROBLEMS

The inventors have conducted intensive studies to solve these problems and have developed a simple method that can concentrate a virus or a viral vector in a solution. More specifically, the inventors have succeeded in preparation of a solution containing a virus having a concentration higher than that of the initial virus-containing solution by binding a lectin to a desired virus-containing solution, binding the resulting virus-lectin complex to a carrier, separating and collecting the carrier to which the virus has been bound, and adding a saccharide to the carrier to elute only the target virus from the carrier into a proper solution without eliminating an infection ability.

The inventors have also found that a recovery rate of a human herpesvirus or its vector is significantly increased by use of a lectin selected from SBA (derived from soybean, binding sugar chain: sugar chain containing N-acetylgalactosamine (GalNAc)), SSA (derived from *Sambucus sieboldiana,* binding sugar chain: sugar chain containing α2,6-linked sialic acid (Siaα2,6)), DSA (derived from *Datura stramonium,* binding sugar chain: sugar chain containing N-acetylglucosamine (GlcNAc)), and WGA (derived from wheat germ, binding sugar chain: sugar chain containing N-acetylglucosamine (GlcNAc) or sialic acid).

The inventors have also found that a recovery rate of a lentiviral vector is significantly increased by use of a lectin selected from WGA and SBA.

Furthermore, the inventors have succeeded in elution of a virus or a viral vector from a carrier to which the virus or viral vector is bound by treating the carrier recovered by the above-mentioned method with a proper saccharide. Surprisingly, the isolated virus maintains its infection ability. The inventors have also found that a preferable saccharide for eluting a virus or a viral vector is a chito-oligosaccharide for WGA or N-acetyl-D-galactosamine or a saccharide containing thereof for SBA.

Based on these findings, the present invention provides a novel method of increasing simply a concentration of a virus or a viral vector while maintaining an infection ability thereof.

Accordingly, the present invention relates to the following aspects.

Embodiment 1: A method of increasing a concentration of a virus, including the steps of:
(1) bringing a lectin into contact with a virus-containing solution to bind the virus to the lectin, wherein (i) the lectin is linked to a molecule capable of binding to a carrier and is immobilized to the carrier through the molecule and thereby makes the virus bound to the lectin bind indirectly to the carrier, or (ii) the lectin is directly immobilized to a carrier and thereby makes the virus bound to the lectin bind to the carrier;
(2) separating the carrier from the virus-containing solution;
(3) treating the recovered carrier with solution containing a saccharide to elute the virus from the carrier, wherein the volume of the eluate is smaller than the volume of the virus-containing solution in step (1); and
(4) separating the carrier from the eluate after step (3) to obtain a concentrated solution of the virus.

Embodiment 2: A method of increasing a concentration of a virus including the steps of:
(1) mixing a virus-containing solution with a biotinylated lectin for bringing a virus into contact with the biotinylated lectin, and then adding a biotin-binding protein-immobilized carrier thereto to make the virus bound to the biotinylated lectin bind to the carrier;
(2) separating the carrier from the virus-containing solution;
(3) treating the recovered carrier with solution containing a saccharide to elute the virus from the carrier, wherein the volume of the eluate is smaller than the volume of the virus-containing solution in step (1); and
(4) separating the carrier from the eluate after step (3) to obtain a concentrated solution of the virus.

Embodiment 3: The method according to embodiment 1 or 2, wherein the virus is a virus having an envelope or a virus derived therefrom.

Embodiment 4: The method according to embodiment 1 or 2, wherein the virus is a lentivirus or a herpesvirus.

Embodiment 5: The method according to embodiment 1 or 2, wherein the lectin is a lectin that binds to a sugar chain containing at least one of N-acetylgalactosamine (GalNAc), α2,6-linked sialic acid (Siaα2,6), and N-acetylglucosamine (GlcNAc).

Embodiment 6: The method according to embodiment 5, wherein the lectin is selected from the group consisting of SBA (derived from soybean), SSA (derived from *Sambucus sieboldiana*), DSA (derived from *Datura stramonium*), and WGA (derived from wheat germ).

Embodiment 7: The method according to embodiment 1 or 2, wherein the lectin is WGA (derived from wheat germ), and the saccharide used for elution of the virus is a chito-oligosaccharide; or the lectin is SBA (derived from soybean), and the saccharide used for elution of the virus is N-acetyl-D-galactosamine or a saccharide containing thereof.

Embodiment 8: The method according to embodiment 1 or 2, wherein the virus is a lentivirus, the lectin is WGA (derived from wheat germ), and the saccharide used for elution of the virus is a chito-oligosaccharide; or the virus is a lentivirus, the lectin is SBA (derived from soybean), and the saccharide used for elution of the virus is N-acetyl-D-galactosamine or a saccharide containing thereof.

Embodiment 9: A method of increasing the concentration of a virus including the steps of:
(1) bringing a virus-containing solution into contact with a lectin being directly bound to nanobeads having a diameter of 10 to 100 nm to make the virus bind to the nanobeads;
(2) separating the nanobeads from the virus-containing solution;
(3) treating the recovered nanobeads with solution containing a saccharide to elute the virus from the nanobeads, wherein the volume of the eluate is smaller than the volume of the virus-containing solution in step (1); and
(4) separating the nanobeads from the eluate after step (3) to obtain a concentrated solution of the virus.

Embodiment 10: A kit for increasing a concentration of a virus by the method according to embodiment 2, the kit including:
a biotinylated lectin, biotin-binding protein-immobilized beads, and a saccharide for eluting the virus from the carrier.

Embodiment 11: A kit for increasing the concentration of a virus by the method according to embodiment 2, the kit including:
(a) biotinylated WGA, a biotin-binding protein, and a chito-oligosaccharide; or
(b) biotinylated SBA, a biotin-binding protein, and N-acetyl-D-galactosamine or a saccharide containing thereof.

### ADVANTAGES OF THE INVENTION

According to the present invention, a virus or a viral vector in a solution can be concentrated readily, while maintaining their infection ability.

### BRIEF DESCRIPTION OF THE DRAWINGS

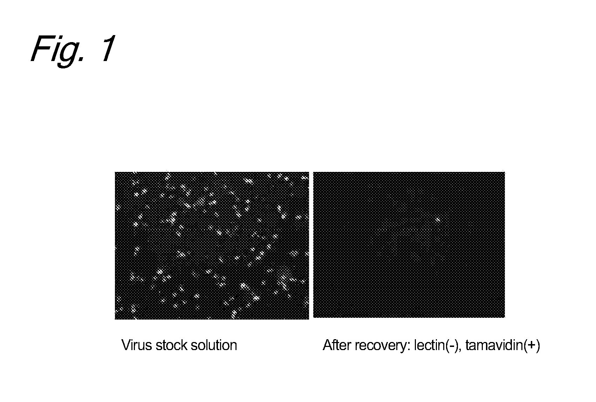

Fig. 1 is fluorescence photomicrographs showing the results of a test for non-specific adsorption of HHV-6 to tamavidin beads. The bright green spots show MT-4 cells serving as indicator cells infected with EGFP recombinant HHV-6. One spot represents one infectious virus particle (the difference in brightness of each spot depends on a difference in gene expression of the virus after infection). The left photograph shows the results after a HHV-6 virus solution (stock solution) was applied to MT-4 cells. The right photograph shows the results after a HHV-6 virus solution (stock solution) was brought into contact with tamavidin beads in the absence of a lectin, and then the virus adsorbed to the tamavidin beads was applied to MT-4 cells.

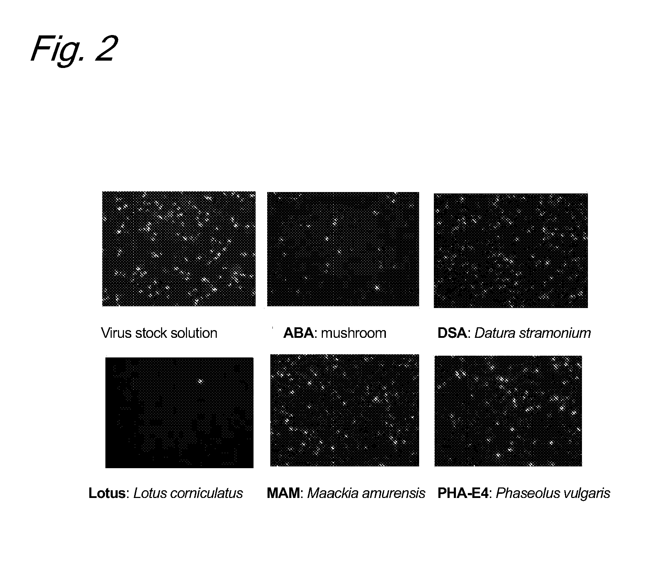

Fig. 2 is fluorescence photomicrographs showing the results of a test for effects of each of lectins, i.e., ABA, DSA, Lotus, MAM, or PHA-E4, on concentration of HHV-6. The bright green spots show MT-4 cells serving as indicator cells infected with EGFP recombinant HHV-6. One spot represents one infectious virus particle (the difference in brightness of each spot depends on a difference in gene expression of the virus after infection).

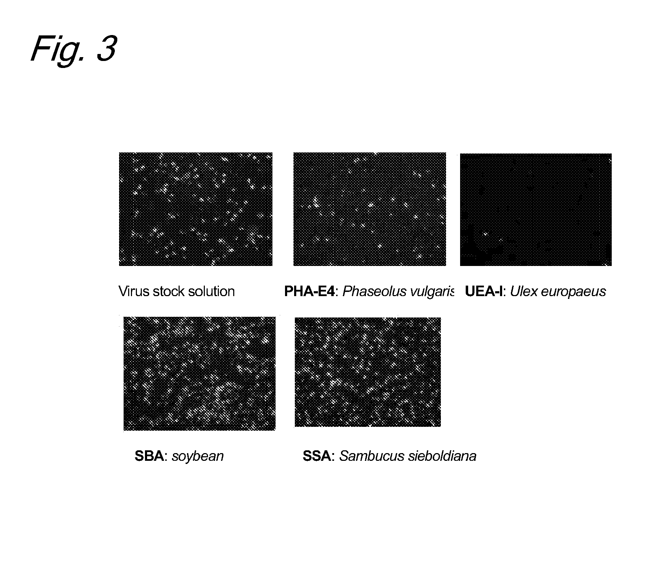

Fig. 3 is fluorescence photomicrographs showing the results of a test for effects of each of lectins, i.e., PHA-L4, UEA-I, SBA, or SSA, on concentration of HHV-6. The bright green spots show MT-4 cells serving as indicator cells infected with EGFP recombinant HHV-6. One spot represents one infectious virus particle (the difference in brightness of each spot depends on a difference in gene expression of the virus after infection).

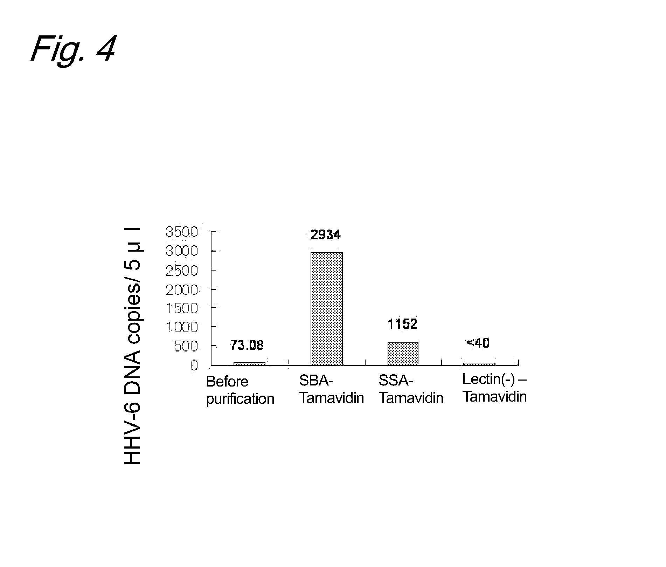

Fig. 4 is a graph showing the degree of enrichment of HHV-6 in saliva by a lectin and tamavidin beads.

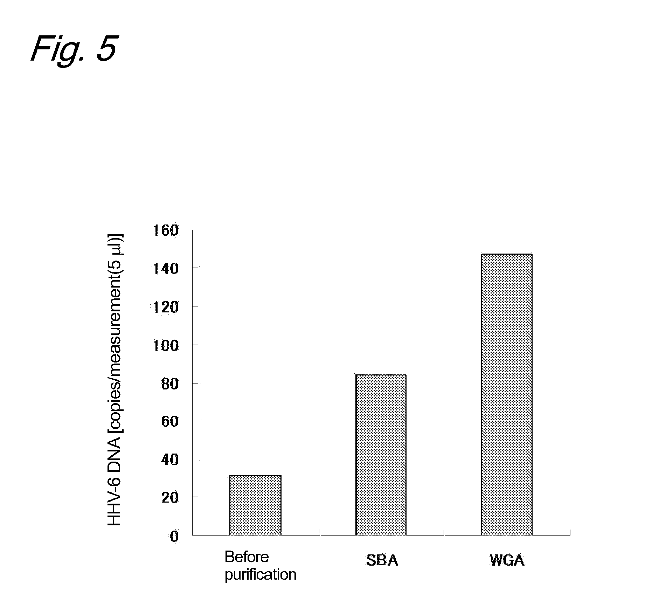

Fig. 5 is a graph showing the degree of enrichment of HHV-6 in saliva by a lectin and tamavidin beads after conducting enrichment of HHV-6 and elution thereof with a saccharide.

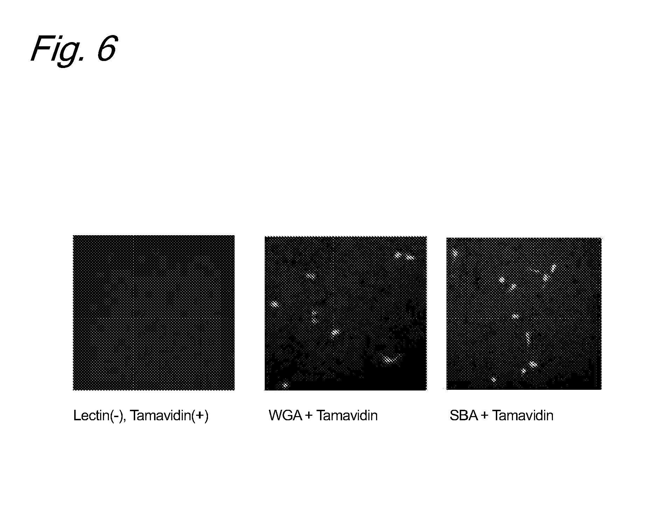

Fig. 6 is fluorescence photomicrographs showing the results of a test for effects of a lectin and tamavidin beads on concentration of a lentiviral vector. The bright green spots show 293 cells serving as indicator cells infected with an EGFP recombinant lentiviral vector. One spot represents one infectious virus particle (the difference in brightness of each spot depends on a difference in gene expression of the virus after infection).

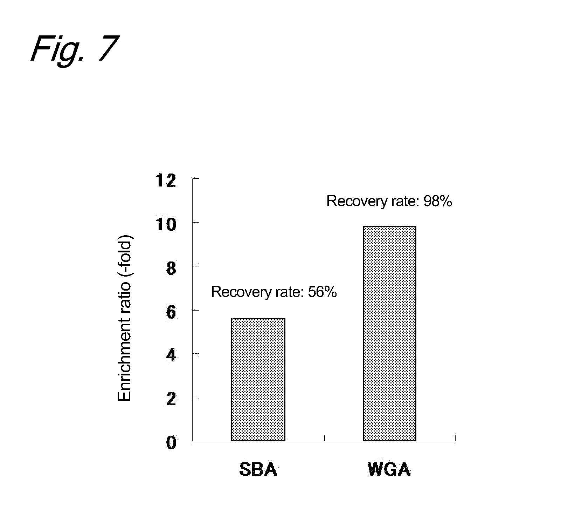

Fig. 7 is a graph showing the degree of enrichment of a lentiviral vector by a lectin and tamavidin beads.

### EMBODIMENTS OF THE INVENTION

The present invention will be described in further detail below.

### 1. Method of concentrating virus

The present invention provides a method of increasing the concentration of a virus, the method including the steps of:
(1) bringing a lectin into contact with a virus-containing solution to bind the virus to the lectin, wherein (i) the lectin is linked to a molecule capable of binding to a carrier and is immobilized to the carrier through the molecule and thereby makes the virus bound to the lectin bind indirectly to the carrier, or (ii) the lectin is directly immobilized to a carrier and thereby makes the virus bound to the lectin bind to the carrier;
(2) separating the carrier from the virus-containing solution;
(3) treating the recovered carrier with solution containing a saccharide to elute the virus from the carrier, wherein the volume of the eluate is smaller than the volume of the virus-containing solution in step (1); and
(4) separating the carrier from the eluate after step (3) to obtain a concentrated solution of the virus.

Each configuration of the method of the present invention will be described in detail below.

### Virus

The virus to be concentrated by the method of the present invention is a virus that has an envelope or a viral vector derived from the virus. In a preferred embodiment, the target virus to be concentrated may be selected from the group consisting of poxviruses, poxvirus-derived vectors, togaviruses, togavirus-derived vectors, coronaviruses, coronavirus-derived vectors, flaviviruses, flavivirus-derived vectors, retroviruses, retrovirus-derived vectors, herpesviruses, and herpesvirus-derived vectors. In another preferred embodiment, the target virus to be concentrated may be a lentivirus, a lentivirus-derived vector, a human herpesvirus 6 (variants A and B of human herpesvirus 6: hereinafter, referred to collectively as HHV-6), a HHV-6-derived vector, a human herpesvirus 7 (hereinafter, referred to as HHV-7), a HHV-7-derived vector, a cytomegalovirus (also called human herpesvirus 5), a HHV-5-derived vector, an Epstein-Barr virus (EBV), or an EBV-derived vector, and more preferably a lentivirus, a lentivirus-derived vector, HHV-6, a HHV-6-derived vector, HHV-7, or a HHV-7-derived vector.

### Virus-containing solution

In this description, the term "virus-containing solution" is a material containing a virus to be concentrated in the method of the present invention. The virus-containing solution may be, for example, a buffer solution containing a virus or a viral vector to be concentrated, or a body fluid. Throughout the description, the term "virus-containing solution" may be referred to simply as a sample.

The solution containing a virus or a viral vector to be concentrated can be prepared by any method known to those skilled in the art.

In this description, the body fluid may be selected from any of saliva, blood, serum, semen, cerebrospinal fluid, pharynx wiping liquid, breast milk, ascitic fluid, perspiration, tear, and urine, without limitation. Body fluid can be collected by any method known to those skilled in the art. The collected body fluid may be treated according to need, as long as the treatment does not affect the infection ability of the virus.

### Lectin

In this description, the term "lectin" refers to a saccharide-binding protein that recognizes a sugar chain and binds thereto. Each lectin selectively binds to specific sugar chains.

The lectin used in the present invention is not limited particularly as long as it can bind to a virus. Preferred examples of the lectin are those that bind to sugar chains containing at least one ofN-acetylgalactosamine (GalNAc), α2,6-linked sialic acid (Siaα2,6), and N-acetylglucosamine (GlcNAc). More preferred examples of the lectin include SBA (a lectin derived from soybean, binding sugar chain: sugar chain containing N-acetylgalactosamine (GalNAc)), SSA (a lectin derived from *Sambucus sieboldiana,* binding sugar chain: sugar chain containing α2,6-linked sialic acid (Siaα2,6)), DSA (a lectin derived from *Datura stramonium,* binding sugar chain: sugar chain containing N-acetylglucosamine (GlcNAc)), and WGA (a lectin derived from wheat germ, binding sugar chain: sugar chain containing N-acetylglucosamine (GlcNAc) or sialic acid).

In particular, SBA, SSA, DSA, or WGA is more preferable for increasing the concentration of a human herpesvirus or a human herpesvirus-derived vector. SBA or WGA is more preferable for increasing the concentration of a lentivirus or a lentivirus-derived vector.

In an embodiment of the present invention, the lectin may be used by being directly immobilized to a carrier.

In another embodiment, the lectin may be used after being indirectly immobilized to a carrier. An example of indirect immobilization is binding of the lectin and the carrier through a binding between biotin and a biotin-binding protein. More specifically, indirect immobilization of the lectin to the carrier can be achieved by biotinylating the lectin and, at the same time, linking the lectin to the surface of the carrier, and then binding the biotin and the biotin-binding protein.

### Carrier

In the present invention, the carrier is not limited particularly as long as it is a solid or insoluble material (for example, a material that can be separated from a reaction mixture by filtration, precipitation, magnetic separation, or other means).

Examples of the material constituting the solid carrier include, but are not limited to, cellulose, Teflon (registered trademark), nitrocellulose, agaroses, highly cross-linked spherical agaroses, dextran, chitosan, polystyrene, polyacrylamide, polyesters, polycarbonates, polyamides, polypropylene, nylons, polydivinylidene difluoride, latex, silica, glass, glass fiber, gold, platinum, silver, copper, iron, stainless steel, ferrite, silicon wafers, polyethylene, polyethyleneimine, polylactic acid, resins, polysaccharides, proteins (e.g., albumin), carbon, and combinations thereof.

Examples of the shape of the solid carrier include, but not limited to, beads, magnetic beads, thin films, microcapillary tubes, filters, plates, microplates, carbon nanotubes, and sensor chips. A planar solid carrier, such as a thin film or a plate, may be provided with, for example, pits, grooves, filter bottoms, as known in the art.

In one embodiment of the present invention, the magnetic beads can have a spherical diameter in the range of from about 10 nm to about 1 mm. In a preferred embodiment, the magnetic beads have a diameter in the ranges of from about 25 nm to about 1 mm and from about 50 nm to about 100 µm. The size of the magnetic beads can be selected according to specific application.

In one embodiment of the present invention, beads made of a highly cross-linked spherical agaroses, such as sepharose, can have a diameter in a range of from about 24 to about 165 µm. In a preferred embodiment, the highly cross-linked spherical agarose beads have a diameter in a range of from about 24 to about 44 µm The size of the highly cross-linked spherical agarose beads can be selected according to specific application.

Examples of the solid carrier having a hydrophobic surface include polystyrene latex beads that are commercially available from Polysciences Inc. or Spherotech Inc.

Examples of silica (SiO₂)-treated or silica (SiO₂)-based solid carrier include superparamagnetic silica beads that are available from Polysciences Inc. In addition, M-280 can be used, which is commercially available from Dynal Biotech LLC.

Examples of the magnetic beads having a hydrophilic surface include beads commercially available from Polysciences Inc. under a trade name of Biomag (registered trademark) carboxyl and beads (MC02N/2928) available from Bangs Laboratory Inc. In addition, M-270 can be used, which is commercially available from Dynal Biotech LLC.

### Direct binding of lectin and carrier

In one embodiment of the present invention, the lectin linked directly to the carrier can be used. In this case, from a viewpoint of increasing a likelihood of association between virus particles and a lectin-immobilized carrier, the carrier should be nanobeads of a size that permits Brownian motion. The preferred spherical diameter of the nanobeads ranges from 10 to 100 nm, more preferably from 30 to 70 nm. In addition, in this case, the nanobeads preferably contain a magnetic material so that the nanobeads can be collected readily with a magnet. Examples of the nanobeads include, but are not limited to, magnetic nanobeads, such as MACS MicroBeads (diameter: 50 nm) available from Miltenyi Biotech Inc.

The lectin and the carrier can be linked by a coupling process between a protein and a carrier known to those skilled in the art. For example, the protein and the carrier can be linked by modifying the carrier surface so as to expose carboxyl groups at the surface and coupling the carboxyl groups to amino groups of the protein in the presence of 1-ethyl-3-(3-dimethylaminopropyl)carbodiimide (EDC), which is a cross-linking reagent. Alternatively, for example, the carboxyl groups of the carrier surface and the amino groups of the protein can be linked by actively esterifying the carboxyl groups of the carrier surface by N-hydroxysuccinimide (NHS) and mixing the carrier with the protein in a buffer that does not contain primary amino groups and has a pH of 6.5 to 9.

Alternatively, the amino groups of the carrier surface and the amino groups of the protein can be linked by using a cross-linking reagent, BS3 (bis[sulfosuccinimidyl] suberate) or DSS (disuccinimidyl suberate); or the amino groups of the carrier surface and the thiol groups of the protein can be linked by using a cross-linking reagent, SPDP (N-succinimidyl 3-[2-pyridyldithio]propionate) or GMBS (N-(4-maleimidebutyryloxy)succinimide).

### Indirect binding of lectin and carrier

In another embodiment of the present invention, the lectin linked indirectly to a carrier may be used. For example, the lectin can be linked indirectly to the carrier, by being biotinylated while a biotin-binding protein is immobilized to the carrier surface and then by being bound to the biotin-binding protein.

In this case, use of beads as a carrier is preferable. A spherical diameter of the beads is preferably in the range of 0.1 to 100 µm, more preferably 0.5 to 10 µm, and most preferably 1 to 5 µm

In this description, the term "biotin" is a general name of D-[(+)-cis-hexahydro-2-oxo-1H-thieno-(3,4)-imidazole-4-valeric acid]. The biotin is a water-soluble vitamin classified into a Vitamin B group and is also called Vitamin B₇, or called Vitamin H or coenzyme R in some cases. The biotin binds to avidin, which is a glycoprotein contained in albumen, with very high affinity.

In this description, the term "biotin" also includes biotin analogs such as iminobiotin (Hofmann, et al., Proc. Natl. Acad. Sci. USA, (1980) 77: 4666-4668), desthiobiotin (Hirsch, et al., Anal. Biochem., (2002) 308: 343-357), biocytin, and biotin sulfoxide, in addition to the above-mentioned biotin.

The lectin may be biotinylated by using a biotinylation reagent. Examples of usable biotinylation reagent include, but not limited to, Pierce products (in parentheses, the linker length and the reactive group are described in this order), such as EZ-Link (registered trademark) Sulfo-NHS-Biotin (13.5 Å, primary amine), EZ-Link (registered trademark) Sulfo-NHS-LC-Biotin (22.4 Å, primary amine), EZ-Link (registered trademark) Sulfo-NHS-LCLC-Biotin (30.5 Å, primary amine), EZ-Link (registered trademark) PFP-Biotin (9.6 Å, amine), EZ-Link (registered trademark) Maleimide-PEO₂-Biotin (29.1 Å, thiol group), EZ-Link (registered trademark) Biotin-PEO₂ Amine (20.4 Å, carboxyl group), EZ-Link (registered trademark) Biotin-PEO₃-LC Amine (22.9 Å, carboxyl group), EZ-Link (registered trademark) Biotin-Hydrazide (15.7 Å, aldehyde group), EZ-Link (registered trademark) Biotin-LC-Hydrazide (24.7 Å, aldehyde group), and EZ-Link (registered trademark) NHS-Iminobiotin (13.5 Å, primary amine).

Using such biotinylation reagent, the lectin can be linked to biotin by a known method.

For example, a biotinylation reagent containing an NHS ester is dissolved in an organic solvent such as DMSO (dimethyl sulfoxide) or a phosphate buffer having pH 7-pH 9, and then the solution is added to the lectin, so that biotin can be linked to the lectin. Alternatively, in a case when a biotinylation reagent containing amino groups is used, biotin may be linked to the lectin by first changing carboxyl groups of the lectin to active ester by using a carbodiimide such as 1-ethyl-3-(3-dimethylaminopropyl)carbodiimide hydrochloride (EDC) and then adding the biotinylation reagent dissolved in a buffer solution having a pH of about 5 to said lectin.

Instead, the biotinylated lectin may be purchased from, for example, J-Oil Mills Inc. as a biotin-labeled lectin. Alternatively, the biotinylated lectin may be produced by linking biotin to a desired lectin by using a biotin labeling kit (for example, though not limited to, EZ-Link (registered trademark) NHS-Lc-Biotin, a product of Pierce Inc.; or Biotin Labeling Kit-NH2, a product of Dojindo Molecular Technologies, Inc.).

Alternatively, for example, a biotinylated lectin may be produced through fusion of a gene of a lectin to a DNA encoding a peptide comprising a biotinylated sequence, construction of a vector expressing this fused gene, and expression of a fusion protein having the biotinylated sequence in a host (Schwarz et al., J. Biol. Chem., (1988), 263: 9640-9645). Examples of the vector include, but not limited to, vectors containing a BioEase (trademark) tag, which is a product of Invitrogen Inc. Among such vectors, a pcDNA (trademark) 6 vector for mammalian cell expression, a pET104 vector for E. coli expression, and a pMT/BioEase vector for Drosophila expression; can be utilized.

In the present invention, any proteins capable of binding to biotin to form biotin/avidin bond, such as avidin, streptavidin, neutravidin, AVR protein (Biochem. J., (2002), 363: 609-617), Bradavidin (J. Biol. Chem., (2005), 280: 13250-13255), Rhizavidin (Biochem. J., (2007), 405: 397-405), tamavidin, and variants thereof, may preferably be used. In particular, tamavidin and its variants can be preferably used. Tamavidin is a biotin-binding protein that was found in basidiomycete Pleurotus cornucopiae, which is an edible mushroom (WO 02/072817; Takakura et al., FEBS J., (2009), 276: 1383-1397). An example of the variant of tamavidin is high affmity/low non-specific binding tamavidin (Japanese Patent Application No. 2008-208766, not yet published).

The term "tamavidin" in the present invention refers to tamavidin 1 (an amino acid sequence: SEQ ID NO: 5, a nucleic acid sequence encoding thereof: SEQ ID NO: 4), tamavidin 2 (an amino acid sequence: SEQ ID NO: 7, a nucleic acid sequence encoding thereof: SEQ ID NO: 6), or a variant thereof. Specifically, the tamavidin of the present invention may be typically a protein comprising the amino acid sequence of SEQ ID NO: 5 or SEQ ID NO: 7 or a protein encoded by a nucleic acid comprising the nucleotide sequence of SEQ ID NO: 4 or SEQ ID NO: 6. Alternatively, the tamavidin of the present invention may be a protein that is a variant of the protein comprising the amino acid sequence of SEQ ID NO: 5 or SEQ ID NO: 7 or the protein encoded by a nucleic acid comprising the nucleotide sequence of SEQ ID NO: 4 or SEQ ID NO: 6 and that has a biotin-binding activity similar to that of tamavidin 1 or 2 or a high affmity/low non-specific binding activity. Throughout the description, tamavidin 1, tamavidin 2, and a variant thereof may be referred to collectively as tamavidin.

The variant of tamavidin 1 or 2 may be a protein containing an amino acid sequence having deletion, substitution, insertion, and/or addition of one or more amino acids in the amino acid sequence of SEQ ID NO: 5 or 7 and having a biotin-binding activity similar to that of tamavidin 1 or 2. The substitution may be a conservative substitution where a specific amino acid residue is substituted by a residue having similar physiochemical characteristics. Nonlimiting examples of the conservative substitution include substitution between amino acid residues having aliphatic groups such as mutual substitution among Ile, Val, Leu, or Ala; and substitution between polar residues such as mutual substitution between Lys and Arg, Glu and Asp, or Gln and Asn.

The variant by deletion, substitution, insertion, and/or addition of amino acid(s) can be produced by, for example, induction of site-specific mutagenesis, which is a well-known technique, (for example, see Nucleic Acid Research, Vol. 10, No. 20, p.6487-6500, 1982, the entire content of which is incorporated in this description by reference), to a DNA encoding a wild-type protein. In this description, the term "one or more amino acids" refers to an amino acid or amino acids that allow deletion, substitution, insertion, and/or addition by the site-specific mutagenesis. In addition, the term "one or more amino acids" in this description may refer to one or several amino acids as appropriate.

Furthermore, the variant of tamavidin 1 or 2 may be a protein comprising an amino acid sequence having an identity of at least 60%, preferably at least 65%, at least 70%, at least 75%, at least 80%, at least 85%, at least 90%, at least 95%, at least 96%, at least 97%, at least 98%, or at least 99%, and more preferably at least 99.3% with the amino acid sequence of SEQ ID NO: 5 or 7 and having a biotin-binding activity similar to that of tamavidin 1 or 2 or a high affmity/low non-specific binding activity.

The identity percentage between two amino acid sequences may be determined by visual inspection and mathematical computation. Alternatively, the identity percentage between two protein sequences may be determined based on the algorithm of Needleman, S. B. and Wunsch, C. D. (J. Mol. Biol., (1970) 48: 443-453) and comparison of sequence information using a GAP computer program, available from the University of Wisconsin Genetics Computer Group (UWGCG). Preferred default parameters of the GAP program include (1) scoring matrix, blosum 62, described in Henikoff, S. and Henikoff, J. G. (Proc. Natl. Acad. Sci. USA, (1992) 89: 10915-10919); (2) a gap weight of 12; (3) a gap length weight of 4; and (4) no penalty for end gap.

Other programs for sequence comparison that are used by those skilled in the art may also be used. The identity percentage can be determined by comparison of sequence information using, for example, the BLAST program described by Altschul, et al. (Nucl. Acids. Res., (1997) 25: 3389-3402). The program is available from the website, National Center for Biotechnology Information (NCBI) or DNA Data Bank of Japan (DDBJ), on the Internet. Each condition (parameter) for identity screening by the BLAST program is shown in the site in detail. Though the conditions may be modified partially, screening generally is performed using default values. Furthermore, the identity percentage between two amino acid sequences may be determined using a program such as the genetic information processing software GENETYX Ver. 7 (Genetyx Company) or FASTA algorithm. In such a case, default values may be used for the screening.

The identity percentage between two nucleic acid sequences can be determined by visual inspection and mathematical computation, or, more preferably, the sequence information is compared using a computer program. A typical and preferred computer program is the Wisconsin package of the genetic computer group (GCG; Wisconsin, Madison), version 10.0 program "GAP" (Devereux, et al., Nucl. Acids Res., (1984) 12: 387). Using this "GAP" program, in addition to comparison of two nucleic acid sequences, comparison of two amino acid sequences and comparison of a nucleic acid sequence and an amino acid sequence can be performed.

In the present invention, examples of preferable tamavidin include the following tamavidin modifications (Japanese Patent Application No. 2008-208766, not yet published):

A modified biotin-binding protein containing an amino acid sequence set forth in SEQ ID NO: 7, or, an amino acid sequence having one or several amino acid modifications in this sequence or an amino acid sequence having an identity of 80% or more with this sequence and showing a biotin-binding activity, wherein one or more residues selected from the following group:
1) an arginine residue at position 104 of SEQ ID NO: 7;
2) a lysine residue at position 141 of SEQ ID NO: 7;
3) a lysine residue at position 26 of SEQ ID NO: 7; and
4) a lysine residue at position 73 of SEQ ID NO: 7,
   are substituted by an acidic or neutral amino acid residue.

More preferably, the modified biotin-binding protein is selected from the group consisting of:
a modified biotin-binding protein (R104E-K141E) where, in SEQ ID NO: 7, the arginine residue at position 104 is substituted by a glutamic acid residue and the lysine residue at position 141 is substituted by a glutamic acid residue;
a modified biotin-binding protein (D40N-R104E) where, in SEQ ID NO: 7, the aspartic acid residue at position 40 is substituted by an asparagine residue and the arginine residue at position 104 is substituted by a glutamic acid residue;
a modified biotin-binding protein (D40N-K141E) where, in SEQ ID NO: 7, the aspartic acid residue at position 40 is substituted by an asparagine residue and the lysine residue at position 141 is substituted by a glutamic acid residue; and
a modified biotin-binding protein (D40N-R104E-K141E) where, in SEQ ID NO: 7, the aspartic acid residue at position 40 is substituted by an asparagine residue, the arginine residue at position 104 is substituted by a glutamic acid residue, and the lysine residue at position 141 is substituted by a glutamic acid residue.

The biotin-binding protein and the carrier can be linked by using a method for coupling a protein and a carrier, which is known to those skilled in the art. For example, the protein and the carrier can be linked by modifying the carrier surface so as to expose carboxyl groups at the surface and coupling the carboxyl groups to amino groups of the protein in the presence of 1-ethyl-3-(3-dimethylaminopropyl)carbodiimide (EDC) serving as a cross-linking reagent. Alternatively, the carboxyl groups of the carrier surface and the amino groups of the protein can be linked by actively esterifying the carboxyl groups of the carrier surface by N-hydroxysuccinimide (NHS) and mixing the carrier with the protein in a buffer that does not contain primary amino groups and has a pH of 6.5 to 9.

Alternatively, amino groups of the carrier surface and amino groups of the protein can be linked by using a cross-linking reagent, BS3 (bis[sulfosuccinimidyl] suberate) or DSS (disuccinimidyl suberate); or amino groups of the carrier surface and thiol groups of the protein can be linked by using a cross-linking reagent, SPDP (N-succinimidyl 3-[2-pyridyldithio]propionate) or GMBS (N-(4-maleimidebutyryloxy)succinimide).

In a case when immobilizing the biotin-binding protein to a carrier, various commercially available carriers having various functional groups at the surfaces can be preferably utilized. Examples of microplates having functional groups on the surfaces include, but are not limited to, maleic anhydride plates such as Reacti-Bind (trademark) Maleic Anhydride Activated Polystyrene 96-Well Plates (Pierce Inc.), active amino group plates such as Immobilizer^{™}-Amino Modules/Plates (Nunc Inc.), and carboxyl group plates such as ELISA plate MS-8796F (96-well C-type/Flat bottom/Carbo) (Sumitomo Bakelite Co., Ltd.). Furthermore, examples of microbeads having a functional group on the surface include, but not limited to, highly cross-linked agarose beads such as Sepharose (trademark) (GE Healthcare Bio-Sciences Ltd.) and magnetic beads such as Dynabeads (trademark) (Dynal Inc.). The biotin-binding protein and the solid carrier can be linked in accordance with instructions attached to the carrier.

Alternatively, examples of the biotin-binding protein-immobilized carrier include, but not limited to, marketed products such as microplates, e.g., Reacti-Bind^{™} Streptavidin Coated Plates (Pierce Inc.) and Nunc Streptavidin Coated 96 Micro Well^{™} Plates (Nalge Nunc Inc.) and magnetic beads, e.g., Dynabeads M-280 Streptavidin (Dynal Inc) and MagnaBind^{™} Streptavidin Beads (Pierce Inc.).

In the case where the biotin or the biotin-binding protein are not used, the lectin and the carrier can also be indirectly bound by, for example, linking the lectin to a histidine tag, a FLAG^{™} tag, or glutathione-S-transferase (GST) and also linking the carrier to the corresponding Ni-NTA (nitrilotriacetic acid), anti-FLAG antibody, and glutathione, and binding the lectin and the carrier.

### Contact of virus-containing solution with lectin

The method of the present invention includes the step of bringing the virus-containing solution into contact with a lectin. The conditions for the contact of the virus-containing solution are not limited particularly as long as the virus in the virus-containing solution binds to the lectin. The factors of the contact conditions include composition of the virus-containing solution, incubation temperature, and incubation time.

The composition of the virus-containing solution is not limited particularly as long as conditions allowing the virus in the virus-containing solution and lectin to bind to each other are maintained. The virus-containing solution is preferably a buffer solution having a pH of 7 to 8, such as a buffer solution based on Tris/EDTA (TE), PBS, HEPES, or TBS. Furthermore, the pH of the virus-containing solution may be 6 to 9, preferably 7 to 8.

When the virus-containing solution is a body fluid, the body fluid may be brought into contact with the lectin directly or after dilution with a desired buffer solution.

The lower limit of the incubation temperature may be 4°C or 10°C, and the upper limit may be selected from the group consisting of 50°C, 45°C, 40°C, 37°C, 30°C, 25°C, and 20°C. For example, the preferred incubation temperature ranges from 10°C to 37°C, from 10°C to 25°C, 10°C to 20°C, or 15°C.

The lower limit of the incubation time may be selected from the group consisting of 1 min, 3 min, 5 min, 10 min, 15 min, 20 min, and 30 min, and the upper limit may be selected from the group consisting of overnight, 10 h, 8 h, 5 h, 3 h, 2 h, 1.5 h, and 1 h. For example, the preferred incubation time ranges from 5 min to overnight, from 5 min to 5 h, and from 10 min to 2 h.

In an embodiment where the lectin is indirectly immobilized to the carrier in the method of the present invention, the lectin and the carrier are brought into contact with and bound to each other through a molecule capable of binding to the carrier, before, while, or after the virus-containing solution comes into contact with the lectin. The conditions for the contact of the lectin and the carrier are not limited particularly as long as the lectin can be sufficiently immobilized to the carrier through the molecule capable of binding to the carrier. The factors of the contact conditions include incubation temperature and incubation time. Conditions similar to those for the contact of the virus and the lectin described above can be selected as appropriate, but a most preferred incubation time ranges from 1 min to 2 h, from 3 min to 1 h, or from 5 to 30 min.

### Separation of carrier

The carrier that has captured the virus to be concentrated through the lectin is separated and recovered from the virus-containing solution being a sample, in a manner known to those skilled in the art according to the properties of the carrier.

For example, in the case of a particulate carrier such as a bead carrier, the carrier may be separated from the sample by centrifugation and subsequent removal of the supernatant. For a carrier that is in a magnetic bead form, the carrier may be separated from the sample by collecting the carrier using a magnet and removing the supernatant. For a carrier that is in a shape of a thin film or filter, the carrier may be separated from the sample by pulling up the carrier from the sample or may be separated from the sample after allowing the sample to pass through the carrier. For a carrier that is designed to capture the virus inside a well structure such as a microplate, the carrier may be separated from the sample merely by removing the sample from the well.

Furthermore, the step of washing the separated carrier may be conducted in order to remove substances non-specifically bound to the carrier that has been separated from the sample, according to need. The conditions of the washing solution and the temperature used in the washing step are not limited particularly as long as the conditions can maintain the binding between the virus and the lectin and/or the binding between the lectin and the carrier.

Preferably, the washing solution is buffered and is a buffer solution having a pH of 7 to 8, such as a buffer solution based on Tris/EDTA (TE), PBS, HEPES, or TBS. The pH of the washing solution may be 6 to 9, preferably 7 to 8.

The washing temperature is not limited particularly, but the lower limit of the washing temperature may be 4°C or 10°C, and the upper limit may be selected from the group consisting of 50°C, 45°C, 40°C, 37°C, 30°C, 25°C, and 20°C. For example, the preferred washing temperature ranges from 10°C to 37°C, from 10°C to 25°C, 10°C to 20°C, or 15°C.

### Elution using saccharides

The method of the present invention includes a step of eluting the virus captured by the carrier through the lectin from the carrier by using solution containing a saccharide. It is presumed that a condition where the virus is bound to a lectin may adversely affect the infection ability of the virus in some types of viruses. Furthermore, in order to concentrate a viral vector to be used in a genetic therapy, it is indispensable to elute and isolate the viral vector from the carrier.

In this description, eluting of virus particles from a carrier refers to that virus particles captured by a carrier are detached from the carrier by dissociating the sugar chainlectin bonds to make the virus particles free from the carrier. Furthermore, in this description, eluting virus particles from a carrier refers to that virus particles captured by a carrier are at least partly detached from the carrier into a free state, but does not necessarily mean that all virus particles captured by the carrier are detached completely.

In this description, the term "isolate" when virus particles are eluted and isolated from a carrier refers to that in which the virus particles captured by the carrier are detached into a free state and are then separated from the carrier component. Accordingly, in this description, the term "isolated virus" refers to that at least the carrier component is not included and does not refer to restriction of the purity of the virus.

The inventors have found that treatment of the virus binding to a lectin with a solution containing a proper saccharide leads to elution and isolation of virus particles from a carrier without eliminating the infection ability of the virus. Accordingly, in the method of the present invention, the virus binding to the lectin can be eluted using a saccharide. The saccharide capable of eluting the virus from the virus-binding lectin is selected from monosaccharides, disaccharides, oligosaccharides, and glycosides that have a saccharide to which the lectin binds.

Specific examples of the saccharide for eluting the virus captured by a lectin include N-acetyl-D-galactosamine specific for SBA, lactose specific for SSA, chito-oligosaccharides specific for DSA, and chito-oligosaccharides and N-acetyl-D-glucosamine specific for WGA, and these can preferably be used.

The solution containing a saccharide may be a buffer solution having a pH of 7 to 8, such as a buffer solution based on Tris/EDTA (TE), PBS, HEPES, or TBS. Furthermore, the pH of the eluate containing a saccharide may be 5 to 9, preferably 7 to 8.

The concentration of a saccharide contained in the eluate containing the saccharide is from 0.01 to 1 M, preferably from 0.05 to 0.5 M, and more preferably from 0.1 to 0.3 M or is from 0.1 to 5% (w/v) and more preferably from 0.5 to 2% (w/v).

The temperature for elution treatment is not limited particularly, and may be room temperature. Alternatively, the lower limit of the washing temperature may be 4°C or 10°C, while the upper limit may be selected from any of 50°C, 45°C, 40°C, 37°C, 30°C, 25°C, and 20°C. For example, the preferred washing temperature is room temperature, from 10 to 37°C, from 10°C to 25°C, 10°C to 20°C, or 15°C.

The time for the elution treatment is not limited particularly, but may be from 30 sec to 1 day, preferably from 5 min to 2 h, and more preferably from 1 to 30 min.

The volume of the eluate used for eluting the virus is not limited particularly as long as the volume is smaller than the initial volume of the virus-containing solution used in the method of the present invention. Preferably, the volume may be 1/2, 1/3, 1/4, 1/5, 1/10, 1/20, 1/50, 1/100, or 1/500 of the initial volume of the virus-containing solution.

In the case where the saccharide is not used in elution from a carrier, the virus can be eluted by adjusting the pH of the eluate to acidic (preferably a pH of 1 to 5) or alkaline (preferably a pH of 9 to 11).

### Preparation of concentrated virus solution

The concentrated virus solution is obtained by removing the carrier, after the carrier is treated with the solution for elusion.

After treatment with the solution , the carrier in the solution is separated and removed from the solution in a manner known to those skilled in the art according to the properties of the carrier. For example, in the case of a particulate carrier such as a bead carrier, the concentrated virus solution may be prepared by centrifugation and subsequent collection of the supernatant. For a carrier that is in a magnetic bead form, the concentrated virus solution may be prepared by collecting the carrier using a magnet and recovering the supernatant. For a carrier that is in a shape of a thin film or filter, the carrier may be separated from the sample by pulling up the carrier from the eluate or may be separated from the eluate after allowing the sample to pass through the carrier. For a carrier that is designed to capture the virus inside a well structure such as a microplate, the concentrated virus solution may be prepared merely by recovering the eluate from the well.

### 2. Kit

The present invention also provides a kit for concentrating the virus in a virus-containing solution based on the method of the present invention.

In an embodiment, the kit of the present invention includes at least a biotinylated lectin, a biotin-binding protein-immobilized carrier, and a saccharide for elution. The lectin, the biotin-binding protein, the carrier, and the saccharide for elution are as defined above.

In another embodiment, the kit of the present invention includes at least lectin-immobilized nanobeads and a saccharide for elution. The lectin, the nanobeads, and the saccharide for elution are as defined above.

The kit of the present invention may further include a buffer solution for binding a virus and a lectin, a buffer solution for washing the separated virus-binding carrier, and/or a buffer solution for eluting the virus from the carrier, in the method of the present invention.

In the kit of the present invention, each reagent may be contained in a proper container. In addition, the kit of the present invention may include a package for properly wrapping the reagents included in the kit.

Furthermore, the kit of the present invention may include suitable instructions for use. Nonlimiting examples of the instructions for use include media that can convey how to use the kit of the present invention to a user, such as description on the package, printed matter, an electronic memory medium (for example, magnetic disk, tape, cartridge, or chip), and an optical medium (for example, CD ROM). In addition, description of the address of an Internet site that provides instructions for use is included in the instructions for use.

### EXAMPLES

The present invention will be described based on examples in more detail, but those examples are not intended to limit the technical scope of the present invention. Those skilled in the art can modify or change the present invention readily based on the description of the present description, and such modifications and changes are included in the technical scope of the present invention.

### Example 1 Screening of lectin that can concentrate HHV-6

Enrichment of HHV-6 was investigated by allowing 15 biotinylated lectins (Con A, DBA, LCA, PHA-E4, PNA, RCA120, UEA-I, WGA, ABA, DSA, Lotus, MAM, PHA-L4, SBA, and SSA) to react with a cultured HHV-6 in solution and then to bind to tamavidin-immobilized magnetic beads.

### 1. Preparation of HHV-6 solution from cultured T cells

Cultured human cord blood-derived T cells were infected with recombinant HHV-6 expressing EGFP (Japanese Patent No. 3923505) to produce an EGFP-type HHV-6 solution.

### 2. Preparation of tamavidin magnetic beads

Magnetic beads (300 µL) having surfaces coated with carboxyl groups (available from Dynabeads M-270 Carboxylic Acid, Dynal Inc.) were washed with 0.01 N sodium hydroxide (300 µL) for 10 min and then with ultrapure water (300 µ) for 10 min three times. To the washed magnetic beads, 1-ethyl-3-(3-dimethylaminopropyl)carbodiimide hydrochloride (EDC) (Pierce Inc.) dissolved in cooled ultrapure water was added into a final concentration of 0.2 M, followed by shaking at room temperature for 30 min. Then, the magnetic beads were washed with cooled ultrapure water (300 µL) and then with 50 mM MES buffer solution (300 µL, pH 5.0), and then mixed with 0.6 mg/mL tamavidin (300 µL, 180 µg) substituted by 50 mM MES buffer solution (pH 5.0). The mixture was shaken at room temperature for 30 min to link the tamavidin and the magnetic beads by covalent bonds. The magnetic beads were collected with a magnet, and the supernatant was removed. Then, unreacted carboxyl groups of the beads were eliminated by 50 mM tris buffer solution (300 µL, pH 7.0), and the magnetic beads were blocked with a PBS buffer solution (300 µL) containing 0.5% BSA and 0.1% Tween 20. The magnetic beads were suspended in a PBS buffer solution (300 µL) to complete the preparation of magnetic beads. Similarly, streptavidin magnetic beads were prepared using streptavidin instead of tamavidin.

### 3. Enrichment of recombinant HHV-6 solution

Using the EGFP-type HHV-6 solution prepared in Section 1, enrichment of HHV-6 was confirmed using the infectious titer of the recombinant HHV-6 as an index. The biotinylated lectins used were the following 15 types: (Con A, DBA, LCA, PHA-E4, PNA, RCA120, UEA-I, WGA, ABA, DSA, Lotus, MAM, PHA-L4, SBA, and SSA) manufactured by J-Oil Mills Inc.

Before the screening of lectins, non-specific adsorption of the tamavidin causing background was confirmed.

First, 100 µL of the EGFP recombinant HHV-6 solution (virus concentration: 10⁴ copies/mL TE) and 500 µL of PBS were mixed and incubated at 15°C for 1 h (upside-down mixing). Then, the tamavidin magnetic beads prepared in Section 2 were added to the reaction solution, followed by incubation at 15°C for 1 h (upside-down mixing). Then, the Eppendorf tube containing the reaction solution was placed in a magnetic stand for Dynabeads, and the beads was washed with 200 µL of PBS containing 2 mM EDTA and 0.5% BSA twice. Then, the beads was suspended in 500 µL of a culture solution (RPMI1640 supplemented with 10% fetal bovine serum), and the whole suspension was mixed with 0.5 mL of an indicator cell (MT4 cell) suspension. It is believed that, if the EGFP-type HHV-6 is present, the indicator cells are infected with the EGFP-type HHV-6, the DNA of the HHV-6 enters the cells, and the EGFP gene incorporated in the HHV-6 is expressed within the indicator cells to generate GFP fluorescence. In this experiment, since the virus particles being bound to the beads and the infected cells both sink in the solution, a decrease in association probability between the virus and the cell, which usually tends to occur, does not occur. Instead, since it is believed that local concentration of the virus is increased significantly, infection efficiency is considered to be improved.

Fig. 1 shows the experimental results confirming that almost no HHV-6 was non-specifically adsorbed to the tamavidin-immobilized beads.

Then, the possibility of increasing concentration of a virus was investigated using each of lectins.

First, 100 µL of the EGFP recombinant HHV-6 solution (virus concentration: 10⁴ copies/mL TE), 500 µL of PBS, and 10 µg of a biotinylated lectin were mixed and incubated at 15°C for 1 h (upside-down mixing). Then, the tamavidin magnetic beads prepared in Section 2 were added to the reaction solution, followed by incubation at 15°C for 1 h (upside-down mixing). Then, the Eppendorf tube containing the reaction solution was placed in a magnetic stand for Dynabeads, and the beads was washed with 200 µL of PBS containing 2 mM EDTA and 0.5% BSA twice. Then, the beads was suspended in 500 µL of a culture solution (RPMI1640 supplemented with 10% fetal bovine serum), and the whole suspension was mixed with 0.5 mL of indicator cell (MT4 cell) suspension. Expression of EGFP was observed. It is believed that, if the EGFP-type HHV-6 is concentrated, the indicator cells are infected with the EGFP-type HHV-6 bound to the magnetic beads through lectin-biotin-tamavidin binding, and the EGFP gene incorporated in the cells is expressed to generate GFP fluorescence.

The experimental results indicated that the expression of EGFP was higher than that of the virus stock solution, when SBA, SSA, DSA, or WGA was used. A part of the results are shown in Figs. 2 and 3. These results suggest that HHV-6 can be efficiently concentrated by the use of these lectins.

### Example 2 Enrichment of HHV-6 in saliva

Enrichment of HHV-6 in saliva was tried using lectinylated biotin and tamavidin magnetic beads.

### 1. Collection of saliva

Saliva was collected from a subject with Salivette (Salivette cotton, Sarstedt). The subject rinsed the oral cavity with distilled water twice immediately before the collection of saliva and put the inner cotton of the Salivette in the oral cavity to collect saliva for 2 min.

### quantitative determination of HHV-6

First, the concentration of HHV-6 in the saliva was measured.

HHV-6 DNA in 400 µL of the saliva collected in Section 1 above was purified using BioRobot EZ1 (Qiagen Inc.) and EZ1 Virus Mini Kit v2.0 (Qiagen Inc.) in accordance with the protocol of EZ1 Virus Mini Handbook (Qiagen Inc.).

The resulting DNA was subjected to quantitative PCR. In the quantitative PCR, the HHV-6 U65/66 region was quantitatively determined by real-time PCR. The sequences used in the PCR were as follows:
Primer: 5'-GACAATCACATGCCTGGATAATG-3' (SEQ ID NO: 1);
Primer : 5'-TGTAAGCGTGTGGTAATGGACTAA-3' (SEQ ID NO: 2); and
TaqMan probe: FAM 5'-AGCAGCTGGCGAAAAGTGCTGTGC-3' TAMRA (SEQ ID NO: 3).
The real-time PCR was performed using the FastStart Universal Probe Master (Rox) (Roche Inc.) at a reaction temperature of 95°C for 10 min once, followed by 45 cycles of 95°C for 5 seconds and 60°C for 31 seconds.

This test was performed by an experimenter highly trained in virus experiments.

The determined HHV-6 DNA in 1 mL of saliva was 14,616 copies/mL. The calculated amount of HHV-6 in 5 µL of saliva is 73.08 copies (Fig. 4: before purification).

### 3. Enrichment of HHV-6 using biotinylated lectin and tamavidin magnetic beads

Saliva (400 µL) collected as in Section 1 were mixed with ten-fold concentration of PBS (44 µL) and 1 nmol of biotinylated SBA, followed by incubation at 15°C for 1 h (upside-down mixing). Then, the tamavidin magnetic beads (100 µL) prepared in Example 1 were added to the reaction solution, followed by further incubation at 15°C for 1 h (upside-down mixing). Then, the Eppendorf tube containing the reaction solution was placed in a magnetic stand for Dynabeads, and the beads was washed with PBS (500 µL) three times. Then, TE (10 µL) was added thereto, followed by incubation at 98°C for 10 min. The supernatant was collected in the magnetic stand, and 5 µL of the supernatant was subjected to quantitative PCR. In the quantitative PCR, the HHV-6 U65/66 region was quantitatively determined by real-time PCR as in Section 2.

The experimental results indicated that the amount of HHV-6 in 5 µL of the eluate subjected to the quantitative PCR was 2934 copies, when the biotinylated SBA and tamavidin magnetic beads were used (Fig. 4). This shows that the HHV-6 solution could be concentrated approximtely 40-fold by using the biotinylated SBA and the tamavidin magnetic beads, compared to the results in Section 2 of this Example. Similarly, the biotinylated SSA gave an approximately 15-fold enrichment ratio. On the other hand, the amount of HHV-6 in the test solution not containining biotinylated lectins was 40 copies or less. Thus, the test solution was not concentrated (Fig. 4).

### 4. Enrichment of HHV-6 using biotinylated lectin and tamavidin magnetic beads and elution by sugar chain

Biotinylated SBA or biotinylated WGA (100 µg) was added to saliva (1 mL) collected as in Section 1, and the tamavidin magnetic beads (50 µL) prepared in Example 1 were added to the reaction solution, followed by upside-down mixing at room temperature for 15 min thereby allowing the components to be bound. The tube containing the reaction solution was placed in a magnetic stand for Dynabeads, and the beads was washed with PBS containing 0.5% bovine serum albumin and 2 mM EDTA twice. Then, the beads was subjected to elution with the solution for elution (20 µL of PBS supplemented with 2% fetal bovine serum and a sugar chain for elution). The saccharide for elution in the solution was N-acetyl-D-galactosamine (0.2 M) in the system using biotinylated SBA and was a chito-oligosaccharide (1%) in the system using the biotinylated WGA. Then, the test tube containing the eluate after the elution treatment was placed in a magnetic stand for Dynabeads, and the tamavidin magnetic beads were separated for recovery of the supernatant. The thus-recovered solution (5 µL) was subjected to quantitative PCR. In the quantitative PCR, as in Section 2, the HHV-6 U65/66 region was quantitatively determined by real-time PCR.

The experimental results indicated that HHV-6 was concentrated 2.7-fold, compared to the virus concentration before purification, by collecting the HHV-6 using the biotinylated SBA and the tamavidin magnetic beads and eluting with N-acetyl-D-galactosamine. Further, HHV-6 was concentrated 4.7-fold, compared to the virus concentration before purification, by collecting the HHV-6 using the biotinylated WGA and the tamavidin magnetic beads and eluting with the chito-oligosaccharide (Fig. 5).

### Example 3 Enrichment of lentiviral vector using lectin

### (1) Enrichment

A lentiviral vector having a HIV-based gene and VSV-G envelope, which is recombined with EGFP gene, were used as a lentivirus (obtained from Dr. Hiroyuki Miyoshi, Riken). The biotinylated lectins used were the following 15 types: (Con A, DBA, LCA, PHA-E4, PNA, RCA120, UEA-I, WGA, ABA, DSA, Lotus, MAM, PHA-L4, SBA, and SSA) manufactured by J-Oil Mills Inc.

First, 100 µL of an EGFP recombinant lentivirus solution (virus concentration: 10² particles/ml TE, note that a low-titer virus was used for investigating enrichment effect) was mixed with 500 µL of PBS and 10 µg of a biotinylated lectin, followed by incubation at 15°C for 1 h (upside-down mixing). Then, the tamavidin magnetic beads prepared in Example 1 were added to the reaction solution, followed by further incubation at 15°C for 1 h (upside-down mixing). Then, the Eppendorf tube containing the reaction solution was placed in a magnetic stand for Dynabeads, and the beads was washed with 200 µL of PBS containing 2 mM EDTA and 0.5% BSA twice. Then, the beads was suspended in 500 µL of a culture solution (RPMI1640 supplemented with 10% fetal bovine serum), and the whole suspension was used for infection of indicator cells (293 cells). The infectious titer of the virus was measured by means of EGFP expression.

The results revealed that the lentiviral vector having the VSV-G envelope was sufficiently concentrated by using WGA or SBA without losing its infection ability (Fig. 6). On the other hand, almost no EGFP expressing cell was observed in the virus of the stock solution.

### (2) Measurement of enrichment ratio

Then, the enrichment ratio was measured. Biotinylated SBA or biotinylated WGA (200 µg) was added to an EGFP recombinant lentivirus solution (1 mL, titer: 10³ particles/mL), and the tamavidin magnetic beads (100 µL) were added to the reaction solution, followed by upside-down mixing at room temperature for 15 min for reaction. The tube containing the reaction solution was placed in a magnetic stand for Dynabeads, and the beads was washed with PBS containing 0.5% bovine serum albumin and 2 mM EDTA twice. Then, the beads was subjected to elution with the solution for elution (100 µL of PBS supplemented with 2% fetal bovine serum and a saccharide for elution). The saccharide for elution in the solution was N-acetyl-D-galactosamine (0.2 M) for the SBA and was a chito-oligosaccharide (1%) for the WGA. Then, the test tube containing the eluate after the elution treatment was placed in a magnetic stand for Dynabeads, and the tamavidin magnetic beads were separated for recovery of the supernatant. The thus-recovered solution was adsorbed to indicator cells (HeLa cells) at 37°C for 1 h for infection. The infected virus titer was determined by counting the number of cells expressing EGFP on next day.

The results show that the recovery rate was 56% in the system using the biotinylated SBA and the recovery rate was 98% in the system using the biotinylated WGA. That is, the enrichment ratios were 5.6 fold and 9.8 fold, respectively (Fig. 7).

### INDUSTRIAL APPLICABILITY

The present invention provides a novel method that can simply increase the virus or viral vector concentration in a solution with a low concentration and a kit for performing the method. The method of the present invention, which can increase the virus or viral vector concentration readily while maintaining the infection ability, is expected to be used, for example, as a safe and simple technique of concentrating a vector useful in a genetic therapy or a vaccine therapy using a viral vector. More specifically, the present invention provides a simple method that does not require a complicated column operation, ultracentrifugation, or other operations, unlike conventional methods, and can be expected to find application in an automated or high-throughput system.

## Claims

1. A method of increasing a concentration of a virus comprising the steps of:
(1) bringing a lectin into contact with a virus-containing solution to bind the virus to the lectin, wherein (i) the lectin is linked to a molecule capable of binding to a carrier and is immobilized to the carrier through the molecule and thereby makes the virus bound to the lectin bind indirectly to the carrier, or (ii) the lectin is directly immobilized to a carrier and thereby makes the virus bound to the lectin bind to the carrier;
(2) separating the carrier from the virus-containing solution;
(3) treating the recovered carrier with solution containing a saccharide to elute the virus from the carrier, wherein the volume of the eluate is smaller than the volume of the virus-containing solution in step (1); and
(4) separating the carrier from the eluate after step (3) to obtain a concentrated solution of the virus.

2. A method of increasing a concentration of a virus comprising the steps of:
(1) mixing a virus-containing solution with a biotinylated lectin for bringing a virus into contact with the biotinylated lectin, and then adding a biotin-binding protein-immobilized carrier thereto to make the virus bound to the biotinylated lectin bind to the carrier;
(2) separating the carrier from the virus-containing solution;
(3) treating the recovered carrier with an solution containing a saccharide to elute the virus from the carrier, wherein the volume of the eluate is smaller than the volume of the virus-containing solution in step (1); and
(4) separating the carrier from the eluate after step (3) to obtain a concentrated solution of the virus.

3. The method according to Claim 1 or 2, wherein the virus is a virus having an envelope or a virus derived therefrom.

4. The method according to Claim 1 or 2, wherein the virus is a lentivirus or a herpesvirus.

5. The method according to Claim 1 or 2, wherein the lectin is a lectin that binds to a sugar chain containing at least one of N-acetylgalactosamine (GalNAc), α2,6-linked sialic acid (Siaα2,6), and N-acetylglucosamine (GlcNAc).

6. The method according to Claim 5, wherein the lectin is selected from the group consisting of SBA (derived from soybean), SSA (derived from *Sambucus sieboldiana*), DSA (derived from *Datura stramonium*), and WGA (derived from wheat germ).

7. The method according to Claim 1 or 2, wherein the lectin is WGA (derived from wheat germ), and the saccharide used for elution of the virus is a chito-oligosaccharide; or the lectin is SBA (derived from soybean), and the saccharide used for elution of the virus is N-acetyl-D-galactosamine or a saccharide containing thereof.

8. The method according to Claim 1 or 2, wherein the virus is a lentivirus, the lectin is WGA (derived from wheat germ), and the saccharide used for elution of the virus is a chito-oligosaccharide; or the virus is a lentivirus, the lectin is SBA (derived from soybean), and the saccharide used for elution of the virus is N-acetyl-D-galactosamine or a saccharide containing thereof.

9. A method of increasing the concentration of a virus comprising the steps of:
(1) bringing a virus-containing solution into contact with a lectin being directly bound to nanobeads having a diameter of 10 to 100 nm to make the virus bind to the nanobeads;
(2) separating the nanobeads from the virus-containing solution;
(3) treating the recovered nanobeads with solution containing a saccharide to elute the virus from the nanobeads, wherein the volume of the eluate is smaller than the volume of the virus-containing solution in step (1); and
(4) separating the nanobeads from the eluate after step (3) to obtain a concentrated solution of the virus.

10. A kit for increasing the concentration of a virus by the method according to Claim 2, the kit comprising:
a biotinylated lectin, biotin-binding protein-immobilized beads, and a saccharide for eluting the virus from the carrier.

11. A kit for increasing the concentration of a virus by the method according to Claim 2, the kit comprising:
(a) biotinylated WGA, a biotin-binding protein, and a chito-oligosaccharide; or
(b) biotinylated SBA, a biotin-binding protein, and N-acetyl-D-galactosamine or a saccharide containing thereof.


SEQUENCE LISTING

<110> Japan Tobacco, Inc

<120> Enrichment method of Virus

<130> P30029-WOEP

<140> 09 727 365.0
<141> 2009-03-31

<150> JP 2008-92816
<151> 2008-03-31

<160> 7

<170> PatentIn version 3.1

<210> 1
<211> 23
<212> DNA
<213> Artificial

<220>
<223> primer

<400> 1
gacaatcaca tgcctggata atg 23

<210> 2
<211> 24
<212> DNA
<213> Artificial

<220>
<223> primer

<400> 2
tgtaagcgtg tggtaatgga ctaa 24

<210> 3
<211> 24
<212> DNA
<213> Artificial

<220>
<223> Taqman probe; FAM is linked to 5'-end, and TAMRA is linked to 3'-
end.

<400> 3
agcagctggc gaaaagtgct gtgc 24

<210> 4
<211> 432
<212> DNA
<213> Pleurotus cornucopiae

<220>
<221> CDS
<222> (1)..(432)
<223> Tamavidin 1

<400> 4
atg aaa gac gtc caa tct ctc ctc acc gga acc tgg tac aat gaa ctc 48
Met Lys Asp Val Gln Ser Leu Leu Thr Gly Thr Trp Tyr Asn Glu Leu

1                   5                   10                  15

ggc tca aca atg aat ttg act gca aat aaa gac ggt tcg ctc acc gga        96
Gly Ser Thr Met Asn Leu Thr Ala Asn Lys Asp Gly Ser Leu Thr Gly
            20                  25                  30

acg tac cac tcc aac gtc ggc gag gtt ccc cca act tat cac ctt tct       144
Thr Tyr His Ser Asn Val Gly Glu Val Pro Pro Thr Tyr His Leu Ser
        35                  40                  45

ggc cgg tac aac ctc cag ccc ccc tcg ggt caa ggc gtt act ctg gga       192
Gly Arg Tyr Asn Leu Gln Pro Pro Ser Gly Gln Gly Val Thr Leu Gly
    50                  55                  60

tgg gcg gtg tct ttc gaa aac act agt gcg aat gtt cat tct gtc tca       240
Trp Ala Val Ser Phe Glu Asn Thr Ser Ala Asn Val His Ser Val Ser
65                  70                  75                  80

aca tgg agc ggg cag tac ttc tct gaa ccc gcc gag gtg atc ctc acc       288
Thr Trp Ser Gly Gln Tyr Phe Ser Glu Pro Ala Glu Val Ile Leu Thr
                85                  90                  95

cag tgg ctg ttg tca agg agc tct gag cgc gaa gat ttg tgg cag tcc       336
Gln Trp Leu Leu Ser Arg Ser Ser Glu Arg Glu Asp Leu Trp Gln Ser
            100                 105                 110

acc cat gtg ggg cat gat gag ttc agc aag aca aag cca acc aag gag       384
Thr His Val Gly His Asp Glu Phe Ser Lys Thr Lys Pro Thr Lys Glu
        115                 120                 125

aag att gcc cag gct caa ctc ctt cgt cgc ggg ttg aag ttc gag tga       432
Lys Ile Ala Gln Ala Gln Leu Leu Arg Arg Gly Leu Lys Phe Glu
    130                 135                 140

<210>  5
<211>  143
<212>  PRT
<213>  Pleurotus cornucopiae

<400>  5

Met Lys Asp Val Gln Ser Leu Leu Thr Gly Thr Trp Tyr Asn Glu Leu
1               5                   10                  15

Gly Ser Thr Met Asn Leu Thr Ala Asn Lys Asp Gly Ser Leu Thr Gly
            20                  25                  30

Thr Tyr His Ser Asn Val Gly Glu Val Pro Pro Thr Tyr His Leu Ser
        35                  40                  45

Gly Arg Tyr Asn Leu Gln Pro Pro Ser Gly Gln Gly Val Thr Leu Gly
    50                  55                  60

Trp Ala Val Ser Phe Glu Asn Thr Ser Ala Asn Val His Ser Val Ser
65                  70                  75                  80

Thr Trp Ser Gly Gln Tyr Phe Ser Glu Pro Ala Glu Val Ile Leu Thr
                85                  90                  95

Gln Trp Leu Leu Ser Arg Ser Ser Glu Arg Glu Asp Leu Trp Gln Ser
            100                 105                 110

Thr His Val Gly His Asp Glu Phe Ser Lys Thr Lys Pro Thr Lys Glu
        115                 120                 125

Lys Ile Ala Gln Ala Gln Leu Leu Arg Arg Gly Leu Lys Phe Glu
    130                 135                 140

<210>  6
<211>  426
<212>  DNA
<213>  Pleurotus cornucopiae

<220>
<221>  CDS
<222>  (1)..(426)
<223>  Tamavidin 2

<400>  6
atg tca gac gtt caa tct tca ctc acc gga acc tgg tac aat gaa ctc        48
Met Ser Asp Val Gln Ser Ser Leu Thr Gly Thr Trp Tyr Asn Glu Leu
1               5                   10                  15

aac tcc aag atg gaa ttg act gca aac aaa gac ggt act ctc act gga        96
Asn Ser Lys Met Glu Leu Thr Ala Asn Lys Asp Gly Thr Leu Thr Gly
            20                  25                  30

aag tac ctc tcc aaa gtt ggg gat gtc tac gtg ccc tac cca ctc tct       144
Lys Tyr Leu Ser Lys Val Gly Asp Val Tyr Val Pro Tyr Pro Leu Ser
        35                  40                  45

ggt cgc tat aac ctc caa ccc ccc gcg gga caa ggc gtc gct ctt ggg       192
Gly Arg Tyr Asn Leu Gln Pro Pro Ala Gly Gln Gly Val Ala Leu Gly
    50                  55                  60

tgg gcg gta tcc tgg gag aac agt aaa att cat tcc gct acg aca tgg       240
Trp Ala Val Ser Trp Glu Asn Ser Lys Ile His Ser Ala Thr Thr Trp
65                  70                  75                  80

agc gga cag ttc ttc tct gag tcg tct cca gtg att ctt act cag tgg       288
Ser Gly Gln Phe Phe Ser Glu Ser Ser Pro Val Ile Leu Thr Gln Trp
                85                  90                  95

ttg ttg tca tcg agc act gcg cgt ggg gac gta tgg gaa tcc aca ctt       336
Leu Leu Ser Ser Ser Thr Ala Arg Gly Asp Val Trp Glu Ser Thr Leu
            100                 105                 110

gtg ggg aat gat tcg ttt aca aag acg gcg ccg act gag cag cag atc       384
Val Gly Asn Asp Ser Phe Thr Lys Thr Ala Pro Thr Glu Gln Gln Ile
        115                 120                 125

gct cat gct caa ctc cat tgt cgc gca ccg agg ttg aag taa               426
Ala His Ala Gln Leu His Cys Arg Ala Pro Arg Leu Lys
    130                 135                 140

<210>  7
<211>  141
<212>  PRT
<213>  Pleurotus cornucopiae

<400>  7

Met Ser Asp Val Gln Ser Ser Leu Thr Gly Thr Trp Tyr Asn Glu Leu
1               5                   10                  15

Asn Ser Lys Met Glu Leu Thr Ala Asn Lys Asp Gly Thr Leu Thr Gly
            20                  25                  30

Lys Tyr Leu Ser Lys Val Gly Asp Val Tyr Val Pro Tyr Pro Leu Ser
        35                  40                  45

Gly Arg Tyr Asn Leu Gln Pro Pro Ala Gly Gln Gly Val Ala Leu Gly
    50                  55                  60

Trp Ala Val Ser Trp Glu Asn Ser Lys Ile His Ser Ala Thr Thr Trp
65                  70                  75                  80

Ser Gly Gln Phe Phe Ser Glu Ser Ser Pro Val Ile Leu Thr Gln Trp
                85                  90                  95

Leu Leu Ser Ser Ser Thr Ala Arg Gly Asp Val Trp Glu Ser Thr Leu
            100                 105                 110

Val Gly Asn Asp Ser Phe Thr Lys Thr Ala Pro Thr Glu Gln Gln Ile
        115                 120                 125

Ala His Ala Gln Leu His Cys Arg Ala Pro Arg Leu Lys
    130                 135                 140

SEQUENCE LISTING

<110> Japan Tobacco, Inc
      Virus Ikagaku Kenkyusho Inc.

<120> Enrichment method of Virus

<130> FA0392-09034

<150> JP 2008-93288
<151> 2008-03-31

<160> 7

<170> PatentIn version 3.1

<210> 1
<211> 23
<212> DNA
<213> Artificial

<220>
<223> primer

<400> 1
gacaatcaca tgcctggata atg                                              23

<210> 2
<211> 24
<212> DNA
<213> Artificial

<220>
<223> primer

<400> 2
tgtaagcgtg tggtaatgga ctaa 24

<210> 3
<211> 24
<212> DNA
<213> Artificial

<220>
<223> Taqman probe; FAM is linked to 5'-end, and TAMRA is linked to 3'-
end.

<400> 3
agcagctggc gaaaagtgct gtgc 24

<210> 4
<211> 432
<212> DNA
<213> Pleurotus cornucopiae

<220>
<221> CDS
<222> (1)..(432)
<223> Tamavidin 1

<400> 4
atg aaa gac gtc caa tct ctc ctc acc gga acc tgg tac aat gaa ctc 48
Met Lys Asp Val Gln Ser Leu Leu Thr Gly Thr Trp Tyr Asn Glu Leu
1               5                   10                  15

ggc tca aca atg aat ttg act gca aat aaa gac ggt tcg ctc acc gga 96
Gly Ser Thr Met Asn Leu Thr Ala Asn Lys Asp Gly Ser Leu Thr Gly
            20                  25                  30

acg tac cac tcc aac gtc ggc gag gtt ccc cca act tat cac ctt tct 144
Thr Tyr His Ser Asn Val Gly Glu Val Pro Pro Thr Tyr His Leu Ser
        35                  40                  45

ggc cgg tac aac ctc cag ccc ccc tcg ggt caa ggc gtt act ctg gga 192
Gly Arg Tyr Asn Leu Gln Pro Pro Ser Gly Gln Gly Val Thr Leu Gly
    50                  55                  60

tgg gcg gtg tct ttc gaa aac act agt gcg aat gtt cat tct gtc tca 240
Trp Ala Val Ser Phe Glu Asn Thr Ser Ala Asn Val His Ser Val Ser
65                  70                  75                  80

aca tgg agc ggg cag tac ttc tct gaa ccc gcc gag gtg atc ctc acc 288
Thr Trp Ser Gly Gln Tyr Phe Ser Glu Pro Ala Glu Val Ile Leu Thr
                85                  90                  95

cag tgg ctg ttg tca agg agc tct gag cgc gaa gat ttg tgg cag tcc 336
Gln Trp Leu Leu Ser Arg Ser Ser Glu Arg Glu Asp Leu Trp Gln Ser
            100                 105                 110

acc cat gtg ggg cat gat gag ttc agc aag aca aag cca acc aag gag 384
Thr His Val Gly His Asp Glu Phe Ser Lys Thr Lys Pro Thr Lys Glu
        115                 120                 125

aag att gcc cag gct caa ctc ctt cgt cgc ggg ttg aag ttc gag tga 432
Lys Ile Ala Gln Ala Gln Leu Leu Arg Arg Gly Leu Lys Phe Glu
    130                 135                 140

<210> 5
<211> 143
<212> PRT
<213> Pleurotus cornucopiae

<400> 5

Met Lys Asp Val Gln Ser Leu Leu Thr Gly Thr Trp Tyr Asn Glu Leu
1               5                   10                  15

Gly Ser Thr Met Asn Leu Thr Ala Asn Lys Asp Gly Ser Leu Thr Gly
            20                  25                  30

Thr Tyr His Ser Asn Val Gly Glu Val Pro Pro Thr Tyr His Leu Ser
        35                  40                  45

Gly Arg Tyr Asn Leu Gln Pro Pro Ser Gly Gln Gly Val Thr Leu Gly
    50                  55                  60

Trp Ala Val Ser Phe Glu Asn Thr Ser Ala Asn Val His Ser Val Ser
65                  70                  75                  80

Thr Trp Ser Gly Gln Tyr Phe Ser Glu Pro Ala Glu Val Ile Leu Thr
                85                  90                  95

Gln Trp Leu Leu Ser Arg Ser Ser Glu Arg Glu Asp Leu Trp Gln Ser
            100                 105                 110

Thr His Val Gly His Asp Glu Phe Ser Lys Thr Lys Pro Thr Lys Glu
        115                 120                 125

Lys Ile Ala Gln Ala Gln Leu Leu Arg Arg Gly Leu Lys Phe Glu
    130                 135                 140

<210> 6
<211> 426
<212> DNA
<213> Pleurotus cornucopiae

<220>
<221> CDS
<222> (1)..(426)
<223> Tamavidin 2

<400> 6
atg tca gac gtt caa tct tca ctc acc gga acc tgg tac aat gaa ctc 48
Met Ser Asp Val Gln Ser Ser Leu Thr Gly Thr Trp Tyr Asn Glu Leu
1               5                   10                  15

aac tcc aag atg gaa ttg act gca aac aaa gac ggt act ctc act gga 96
Asn Ser Lys Met Glu Leu Thr Ala Asn Lys Asp Gly Thr Leu Thr Gly
            20                  25                  30

aag tac ctc tcc aaa gtt ggg gat gtc tac gtg ccc tac cca ctc tct 144
Lys Tyr Leu Ser Lys Val Gly Asp Val Tyr Val Pro Tyr Pro Leu Ser
        35                  40                  45

ggt cgc tat aac ctc caa ccc ccc gcg gga caa ggc gtc gct ctt ggg 192
Gly Arg Tyr Asn Leu Gln Pro Pro Ala Gly Gln Gly Val Ala Leu Gly
    50                  55                  60

tgg gcg gta tcc tgg gag aac agt aaa att cat tcc gct acg aca tgg 240
Trp Ala Val Ser Trp Glu Asn Ser Lys Ile His Ser Ala Thr Thr Trp
65                  70                  75                  80

agc gga cag ttc ttc tct gag tcg tct cca gtg att ctt act cag tgg 288
Ser Gly Gln Phe Phe Ser Glu Ser Ser Pro Val Ile Leu Thr Gln Trp
                85                  90                  95

ttg ttg tca tcg agc act gcg cgt ggg gac gta tgg gaa tcc aca ctt 336
Leu Leu Ser Ser Ser Thr Ala Arg Gly Asp Val Trp Glu Ser Thr Leu
            100                 105                 110

gtg ggg aat gat tcg ttt aca aag acg gcg ccg act gag cag cag atc 384
Val Gly Asn Asp Ser Phe Thr Lys Thr Ala Pro Thr Glu Gln Gln Ile
        115                 120                 125

gct cat gct caa ctc cat tgt cgc gca ccg agg ttg aag taa 426
Ala His Ala Gln Leu His Cys Arg Ala Pro Arg Leu Lys
    130                 135                 140

<210> 7
<211> 141
<212> PRT
<213> Pleurotus cornucopiae

<400> 7

Met Ser Asp Val Gln Ser Ser Leu Thr Gly Thr Trp Tyr Asn Glu Leu
1               5                   10                  15

Asn Ser Lys Met Glu Leu Thr Ala Asn Lys Asp Gly Thr Leu Thr Gly
            20                  25                  30

Lys Tyr Leu Ser Lys Val Gly Asp Val Tyr Val Pro Tyr Pro Leu Ser
        35                  40                  45

Gly Arg Tyr Asn Leu Gln Pro Pro Ala Gly Gln Gly Val Ala Leu Gly
    50                  55                  60

Trp Ala Val Ser Trp Glu Asn Ser Lys Ile His Ser Ala Thr Thr Trp
65                  70                  75                  80

Ser Gly Gln Phe Phe Ser Glu Ser Ser Pro Val Ile Leu Thr Gln Trp
                85                  90                  95

Leu Leu Ser Ser Ser Thr Ala Arg Gly Asp Val Trp Glu Ser Thr Leu
            100                 105                 110

Val Gly Asn Asp Ser Phe Thr Lys Thr Ala Pro Thr Glu Gln Gln Ile
        115                 120                 125

Ala His Ala Gln Leu His Cys Arg Ala Pro Arg Leu Lys
    130                 135                 140